# EUROPEAN PATENT SPECIFICATION

(11) **EP 2 632 353 B1**
(45) Date of publication and mention of the grant of the patent: **29.03.2017**
(21) Application number: 11778982.6
(22) Date of filing: 19.10.2011
(51) Int. Cl.: A61B 17/70

(54) **LAMINOPLASTY IMPLANT, IN PARTICULAR FOR CERVICAL LAMINOPLASTY**
IMPLANTAT FÜR DIE LAMINOPLASTIE, INSBESONDERE FÜR DIE ZERVIKALE LAMINOPLASTIE
IMPLANT DE LAMINOPLASTIE, EN PARTICULIER POUR LAMINOPLASTIE CERVICALE

(30) Priority: 28.10.2010 FR 1058911
(43) Date of publication of application: 04.09.2013
(73) Proprietor: Medicrea International, 69140 Rillieux-la-Pape (FR)
(72) Inventor: PASCAL MOUSSELARD, Hugues, F-75015 Paris (FR)
(74) Representative: Jeannet, Olivier
(86) International application number: PCT/IB2011/054669
(87) International publication number: WO 2012/056375

(56) References cited:
- CN-A- 101 785 694
- CN-U- 201 453 355
- FR-A1- 2 918 262
- US-A1- 2003 045 936
- US-A1- 2004 030 388

## Description

The present invention relates to a laminoplasty implant, in particular for cervical laminoplasty.

It is known to treat a compression of the spinal cord at the cervical vertebrae through a so-called "laminoplasty" operation, consisting of enlarging the vertebral canal.

One known laminoplasty technique consists of cutting a lateral zone of the laminae of one or more vertebrae, then producing outward pivoting of one or two portions of lamina defined by said cutting, and putting an implant in place between these portions of lamina in order to keep the latter in that pivoted position. An implant adapted to this technique is described by documents US 2004/030388 A1 (figures 4 to 7) or CN 101 785 694 A.

Another known laminoplasty technique, called "double door," consists of performing an ablation of the central zone of the lamina, producing two lateral grooves at the base of the portions of lamina thus formed, on the outer side, both to reduce the thickness of the bone until its flexibility is allowed and to produce a recess making it possible to pivot each portion of lamina toward the outside, pivoting each portion of lamina toward the outside, and lastly placing an implant on the portions of lamina. One implant adapted to this technique is described by document CN 201 453 355 U or US 2003/045936 A1.

Still another known laminoplasty technique consists of performing an ablation of the central zone of the lamina, and then connecting the two portions of lamina thus formed using an implant. One known implant adapted to the latter technique is disclosed by documents US 2004/030388 A1 (figures 20 to 22) or FR 2 918 262 A1. Such an implant comprises a central body in the form of a strip and lateral ends in the form of U clips, the bottom walls of said U clips being situated on the outer side of the implant and, when implanted, covering the resected surfaces of free ends of the portions of lamina.

An implant of this type has the drawback of not being very secure for the practitioner to place in light of the risk of lesion of the spinal cord during impacting of said clips on the inner and outer faces of the portions of lamina. Furthermore, these existing implants do not allow perfect reconstruction of the bone bridge naturally formed by the posterior arc of a vertebra, nor, as a result, complete reinsertion of the soft tissues normally anchored on a lamina or a spinous process.

The present invention aims to resolve these drawbacks.

The implant it concerns is adapted to connect the two portions of the lamina of a vertebra resulting from an ablation of the central zone of said lamina, and to that end comprises, in a known manner:
- a body having a first end intended to be connected to a first portion of lamina and a second end intended to be connected to the second portion of lamina, said body having, in its implantation position, an outer side, an inner side, an upper side and a lower side; a first direction extends from the outer side toward the inner side of said body; and
- two anchoring portions to said portions of lamina, one of which is connected to said first end and the other of which is connected to said second end, each anchoring portion being in the shape of a U, i.e. comprising an inner side wall intended to come against an inner side of the corresponding portion of lamina, an outer side wall intended to come against an outer side of said portion of lamina, and an intermediate wall connecting said inner side wall to said outer side wall; a second direction extends from said intermediate wall, substantially perpendicularly thereto, toward the opening of the U defined between the free end edges of said inner side wall and said outer side wall.

According to the invention,
- said intermediate wall of each anchor portion is situated toward the upper side of the body of the implant, and said second direction is substantially perpendicular to said first direction, said inner, outer and intermediate side walls thus being arranged relative to said body so that, when the body is arranged in said implantation position, the direction of engagement of these walls on the portions of lamina is parallel to the cranial-caudal direction of the vertebral column, i.e. the general longitudinal direction of said vertebral column; and
- said central body comprises mounting means allowing one or more grafts to be, mounted thereon so as to extend from one resected surface of a portion of lamina to the resected surface of the other portion of lamina, the ends of that or those grafts being thus situated, after placement, in the immediate vicinity of said resected surfaces.

Thus, the implant according to the invention comprises side and intermediate walls whereof the engagement on the portions of lamina is done in the cranial-caudal direction, i.e. parallel to the general longitudinal direction of the vertebral column. This engagement is done using a much safer process, in terms of risks of lesion of the spinal cord, than the engagement of an implant according to the prior art, done in the antero-posterior direction, i.e. perpendicular to the general longitudinal direction of the vertebral column. Moreover and above all, this structure makes it possible to leave the resected surfaces of the portions of lamina free and therefore makes it possible to place one or more grafts extending from one portion of lamina to the other as far as the vicinity of said resected surfaces. The reconstruction of a bone bridge between the portions of lamina can then be done under better conditions, by growth of the bone cells of the portions of lamina toward the graft(s). This bone bridge makes it possible to perform a perfect reinsertion of the soft tissues normally anchored on a lamina or a spinous process.

Furthermore, said intermediate walls, owing to their bearing against the portions of lamina, constitute stop surfaces making it possible to completely stabilize the implant in said implantation position.

Advantageously, each intermediate wall is deformable between a first position, in which the outer side wall to which said intermediate wall is connected is remote from the inner side wall to which said intermediate wall is connected, and a second position, in which said outer side wall is closer to said inner side wall so as to grip a portion of lamina between it and said inner side wall.

Said first position is an insertion position of the implant, in which the outer side wall is moved away from the corresponding inner side wall, which greatly facilitates the insertion of the implant on a vertebra. Once said implantation position is reached, the intermediate walls are deformed so as to fold the outer side walls down against the portions of lamina, in said second position, and to thereby grip the portions of lamina between said outer and inner walls, thereby ensuring fastening of the implant.

The implant according to the invention thus has still further increased safety relative to the risk of lesion of the patient's spinal cord during placement of said implant, no impaction being necessary to insert, more or less forcibly, the clips on said portions of lamina, as is the case for the implants according to the prior art.

Preferably, each inner side wall extends in a direction forming, with the inner side of the body of the implant, an angle going from 100 to 120°. This angle is preferably 110°.

The two side walls, as well as the intermediate walls connected to them, thus diverge from one another in the direction moving away from said inner side of the body. This shape is completely adapted to the shape of the cut out, and potentially pivoted, portions of lamina.

Each side wall and/or each intermediate wall can comprise means favouring its fastening relative to a portion of lamina, in particular sharp teeth that can be inserted into the bone of said portion of lamina, and/or one or more holes making it possible to place screws through said portion of lamina.

Preferably, said mounting means comprised by said central body for assembling one or more grafts are in the form of a cage having a transverse conduit, the ends of which emerge laterally.

This transverse conduit allows easy placement of the graft(s) on the implant, and perfect maintenance of said graft(s).

Said case can form a single piece with said body, or can be formed by a piece separate from said body and fixed thereon. In the first case, the implant assembly can in particular be made from a biocompatible material, in particular titanium; in the second case, said body and said side and intermediate walls are made from a piece of titanium while said cage is made from a synthetic material, in particular PEEK (polyetheretherketone). The two-piece version offers more possibilities as to the shapes the cage can be given.

Said cage can assume a flat triangular shape, similar to that of a spinous process, and thus allow an at least approximate reconstitution of said spinous process when the latter is resected.

According to one preferred embodiment of the invention, in that case, said cage has concave lateral edges and rounded corners. It can also have, seen in a transverse direction, a height that gets smaller on the side opposite said body.

The invention will be well understood, and other features and advantages thereof will appear, in reference to the appended diagrammatic drawing, showing, as nonlimiting examples, two embodiments of the concerned laminoplasty implant.
Figure 1 is a perspective view, according to a first embodiment, before assembly;
figure 2 is a view similar to figure 1, after assembly;
figure 3 is a top view, before assembly;
figure 4 is a side view;
figure 5 is a front view, posterior side, before placement on a vertebra whereof the central portion of the lamina has been resected and the portions of lamina of which generated by said resection have been pivoted outwardly;
figure 6 is a view similar to figure 5, during placement on the vertebra;
figure 7 is a view similar to figure 6, during fastening on the vertebra;
figure 8 is a perspective view, according to a second embodiment, and
figure 9 is a view similar to figure 8, before placement on a vertebra whereof the central portion of the lamina has been resected and the portions of lamina of which generated by said resection have been pivoted outwardly.

For simplification purposes, the elements or parts of the implant according to the first embodiment that are found identically or similarly on the implant according to the second embodiment will be designated using the same numerical references and will not be described again.

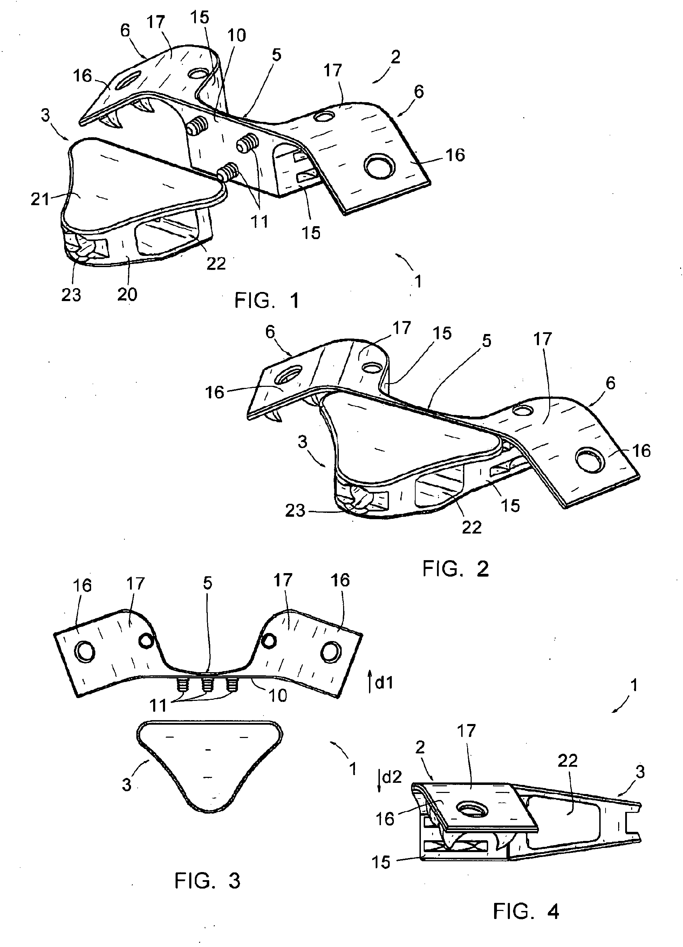

Figures 1 to 4 show a laminoplasty implant 1, in particular for cervical laminoplasty, that comprises a base piece 2 made from a biocompatible metal material, in particular titanium, and a cage 3 made from a synthetic material, in particular PEEK (polyetheretherketone).

The base piece 2 forms a body 5 and two laminar anchoring side portions 6.

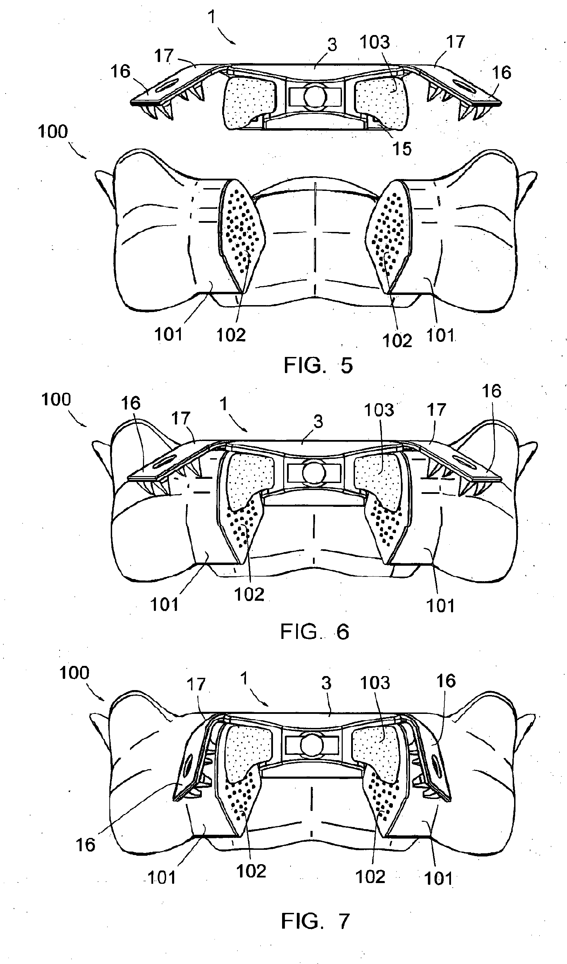

The body 5 is formed by a wall 10 that can, in an implantation position (see figures 6 and 7), connect the two portions 101 of a cervical vertebral lamina resulting from an ablation of the central portion of said lamina. The inner face of said wall 10, intended to face the vertebral canal, is smooth, while the outer surface of said wall comprises two lugs 11 with sharp skirts, for assembling the cage 3. A direction d1 extending from the outer side to the inner side of the body 5 (see figure 3) is defined as a "first direction".

Each laminar anchoring side portion 6 comprises an inner side wall 15, an outer side wall 16 and an intermediate wall 17 connecting the wall 15 to the wall 16 so that each anchoring portion 6 is in the shape of a U. A direction extending from the intermediate wall 17, substantially perpendicular thereto, toward the opening of the U defined between the free end edges of the side walls 15 and 16 (see figure 4) is defined as a "second direction".

Each inner side wall 15 is adjacent to the corresponding end of the body 5, with which it forms a body, and includes sharp teeth protruding from its outer surface, i.e. its surface facing the corresponding outer side wall, in particular four teeth arranged in a square as in the illustrated example. As shown in figure 3, the side 15 and intermediate 17 walls extend in a direction forming, with the inner surface of the body 5, a substantially 110° angle.

Each outer side wall 16 is connected to the corresponding intermediate support wall 17. It is passed through by a hole for receiving a screw allowing it to be fixed to the portion of lamina 101 and includes sharp teeth, with a curved shape, protruding from its inner surface, i.e. its surface facing the corresponding inner side wall 15, in particular four teeth arranged in a square as in the illustrated example.

Each intermediate support wall 17 is adjacent, on two opposite edges, to the corresponding inner side wall 15 and outer side wall 16, respectively. It also comprises a hole for receiving a screw allowing it to be fixed to the portion of lamina 101. Each intermediate wall 17 is also deformable non-elastically between an insertion position of the implant, shown in figures 1 to 6, in which each outer side wall 16 is moved away from the corresponding inner side wall 15, and a fixing position of the implant 1, shown in figure 7, in which said outer side wall 16 is brought closer to the corresponding inner side wall 15 so as to grip the portion of lamina 101 between it and said inner side wall 15, with penetration of the teeth of said side walls into the bone of the portion of lamina 101.

In particular, in the illustrated example, the wall 16 and the wall 17 of each anchoring portion are formed by a same flexible material, the wall 16 being in the continuous extension of the wall 17.

As shown clearly in figures 3 and 4, said second direction d2 is substantially perpendicular to said first direction d1.

The cage 3 has a flat triangular shape, similar to that of a spinous process, and at a height that gets smaller on the side opposite said body 5 (see figure 4). It comprises a body 20 and an upper wall 21 with a triangular shape, with concave side edges and rounded corners. The body 20 forms a transverse conduit 22 whereof the ends emerge laterally, and comprises an anterior wall pierced with holes for forcibly receiving lugs 11 and a posterior wall having a cavity 23 for receiving a handling instrument.

As understood in reference to figures 1 and 2, the implant 1 is formed by assembling the cage 3 to the base piece 2, said assembly being done by forcible insertion of the lugs into the holes comprised by the anterior wall of the cage 3.

In practice, as shown in figures 5 to 7, one or more cervical vertebrae 100 are the subject of an ablation of the central zone of the laminae, thereby individualizing, on a vertebra, two portions of lamina 101 with resected end surfaces 102.

One or more grafts 103, having lengths such that their ends can be situated, after placement of the implant 1, in the immediate vicinity of the resected surfaces 102, are placed through the conduit of the cage 3, then the implant 1 is inserted toward its implantation side on the vertebra 100 (see figure 5). As will be understood, this insertion is done in a cranial-caudal direction, i.e. parallel to the general longitudinal direction of the vertebral column, and continues until engagement of the inner side walls 15 on the inner side surfaces of the portions of lamina 101 and with the intermediate walls 17 bearing against the upper edges of said portions of lamina 101 (see figure 6). In that position, the ends of the graft(s) come into intimate contact with the resected surfaces 102.

The intermediate walls 17 are then deformed so as to fold the outer side walls 16 down against the outer surfaces of the portions of lamina 101 (see figure 7), and, in so doing, to insert the teeth of the side walls into the bone of the portions of lamina 101. Fastening screws (not shown) can then be placed through the holes comprised by said walls.

The implant 1 according to the invention thus comprises laminar anchoring side portions 6 whereof the engagement on the portions of lamina 101 is done in the cranial-caudal direction, using a much safer process, in terms of risk of lesion of the spinal cord, than the engagement of an implant according to the prior art, done in the antero-posterior direction, i.e. perpendicular to the general longitudinal direction of the vertebral column. Furthermore and above all, the implant 1 makes it possible to leave the resected surfaces 102 free and therefore to place one or more grafts 103 extending from one portion of lamina 101 to the other up to the vicinity of these resected surfaces 102. The reconstruction of a bone bridge between these portions of lamina 101 can then be done under the best possible conditions, by bone cell growth of the portions of lamina 101 toward the graft(s) 103. This bone bridge makes it possible to perform perfect reinsertion of the soft tissues normally anchored on a lamina or on a spinous process of a vertebra 100.

In the implantation position, the intermediate walls 17 bear against the upper edges of the portions of lamina 101 and thus form stop and stabilization surfaces of the implant 1.

The placement of the implant 1 is made easier and safer by spacing the outer side walls 16 apart from the inner walls 15; once they are folded down against the outer surfaces of the portions of lamina, said outer walls 16, jointly with the inner walls 16, make it possible to ensure complete fixing of the implant 1 to the portions of lamina 101.

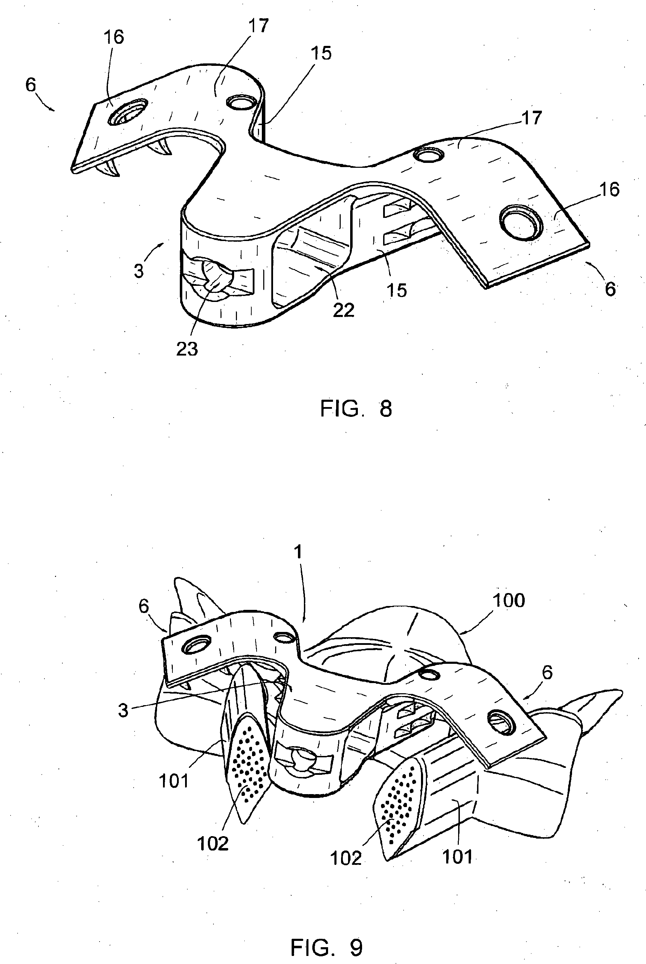

The second embodiment of the implant 1, shown in figures 8 and 9, is very similar to the first embodiment described above; it differs from the latter only in that the cage 3 forms a body with the base piece 2. Said cage 3 is therefore made from the same metallic material as the base piece 2.

The invention was described above in reference to embodiments provided as examples. It of course is not limited to those embodiments and extends to all other embodiments covered by the appended claims.

## Claims

1. Laminoplasty implant (1), in particular for cervical laminoplasty, adapted to connect the two portions of the lamina (101) of a vertebra resulting from an ablation of the central zone of said lamina, comprising:
- a body (5) having a first end and a second end, said body (5) having an outer side, an inner side, an upper side and a lower side; a first direction (d1) extends from the outer side toward the inner side of said body (5); and
- two anchoring portions (6), one of which is connected to said first end and the other of which is connected to said second end, each anchoring portion (6) being in the shape of a U, i.e. comprising an inner side wall (15), an outer side wall (16), and an intermediate wall (17) connecting said inner side wall (15) to said outer side wall (16); a second directibn (d2) extends from said intermediate wall (17), perpendicularly thereto, toward the opening of the U defined between the free end edges of said inner side wall (15) and said outer side wall (16);
- said body (5) comprises mounting means (3);
**characterized in that** said intermediate wall (17) of each anchor portion (6) is situated at the upper side of the body (5) of the implant, and said second direction (d2) is perpendicular to said first direction (d1).

2. Laminoplasty implant (1) according to claim 1, **characterized in that** each intermediate wall (17) is deformable between a first position, in which the outer side wall (16) to which said intermediate wall (17) is connected is remote from the inner side wall (15) to which said intermediate wall (17) is connected, and a second position, in which said outer side wall (16) is closer to said inner side wall (15).

3. Laminoplasty implant (1) according to claim 1 or claim 2, **characterized in that** each inner side wall (15) extends in a direction forming, with the inner side of the body (5) of the implant, an angle going from 100 to 120°.

4. Laminoplasty implant (1) according to claim 3, **characterized in that** each inner side wall (15) extends in a direction forming, with the inner side of the body (5) of the implant, an angle of 110°.

5. Laminoplasty implant (1) according to anyone of claims 1-4, **characterized in that** each side wall (15, 16) and/or each intermediate wall (17) comprise means favouring its fastening relative to a portion of lamina (101).

6. Laminoplasty implant (1) according to claim 5, **characterized in that** said means favouring the fastening relative to a portion of lamina (101) are sharp teeth that are able to be inserted into the bone of said portion of lamina (101), and/or one or more holes making it possible to place screws through said portion of lamina (101).

7. Laminoplasty implant (1) according to anyone of claims 1-6, **characterized in that** said mounting means (3) comprised by said central body (5) for assembling one or more grafts (103) are in the form of a cage (3) having a transverse conduit (22), the ends of which emerge laterally.

8. Laminoplasty implant (1) according to claim 7, **characterized in that** the cage (3) assumes a flat triangular shape.

9. Laminoplasty implant (1) according to claim 8, **characterized in that** the cage (3) has concave lateral edges and rounded corners, and, seen in a transverse direction, a height that gets smaller on the side opposite said body (5).

## Patentansprüche

1. Implantat (1) für die Laminoplastie, insbesondere für die zervikale Laminoplastie, angepasst, die zwei Abschnitte der Lamina (101) eines Wirbels zu verbinden, die durch eine Ablation des zentralen Bereichs der Lamina entstehen, umfassend: :
- einen Körper (5), der ein erstes Ende und ein zweites Ende aufweist, wobei der Körper (5) eine Außenseite, eine Innenseite, eine Oberseite und einer Unterseite aufweist; eine erste Richtung (d1) erstreckt sich von der Außenseite zur Innenseite des Körpers (5); und
- zwei Ankerabschnitte (6), von denen der eine mit dem ersten Ende und der andere mit dem zweiten Ende verbunden ist; wobei jeder Ankerabschnitt (6) eine U-Form aufweist, d. h. eine Innenseitenwand (15), eine Außenseitenwand (16) und eine Zwischenwand (17) umfasst, die die Innenseitenwand (15) mit der Außenseitenwand (16) verbindet; eine zweite Richtung (d2) erstreckt sich von der Zwischenwand (17), senkrecht dazu, hin zur Öffnung des U, die zwischen den freien Endkanten der Innenseitenwand (15) und der Außenseitenwand (16) bestimmt ist;
- wobei der Körper (5) Einbaumittel (3) umfasst;
**dadurch gekennzeichnet, dass** die Zwischenwand (17) jedes Ankerabschnitts (6) an der Oberseite des Körpers (5) des Implantats angeordnet ist und dass die zweite Richtung (d2) senkrecht zur ersten Richtung (d1) ist.

2. Implantat (1) für die Laminoplastie nach Anspruch 1, **dadurch gekennzeichnet, dass** jede Zwischenwand (17) zwischen einer ersten Position, in der die Außenseitenwand (16), mit der die Zwischenwand (17) verbunden ist, von der Innenseitenwand (15), mit der die Zwischenwand (17) verbunden ist, entfernt ist, und einer zweiten Position verformbar ist, in der die Außenseitenwand (16) näher an der Innenseitenwand (15) ist.

3. Implantat (1) für die Laminoplastie nach Anspruch 1 oder 2, **dadurch gekennzeichnet, dass** sich jede Innenseitenwand (15) in eine Richtung erstreckt, die mit der Innenseite des Körpers (5) des Implantats einen Winkel von 100 bis 120° bildet.

4. Implantat (1) für die Laminoplastie nach Anspruch 3, **dadurch gekennzeichnet, dass** sich jede Innenseitenwand (15) in eine Richtung erstreckt, die mit der Innenseite des Körpers (5) des Implantats einen Winkel von 110° bildet.

5. Implantat (1) für die Laminoplastie nach einem der Ansprüche 1 bis 4, **dadurch gekennzeichnet, dass** jede Seitenwand (15, 16) und/oder jede Zwischenwand (17) Mittel umfasst, die ihre Befestigung hinsichtlich eines Abschnitts der Lamina (101) begünstigen.

6. Implantat (1) für die Laminoplastie nach Anspruch 5, **dadurch gekennzeichnet, dass** die Mittel, die die Befestigung hinsichtlich eines Abschnitts der Lamina (101) begünstigen, scharfe Zähne sind, die in der Lage sind, in den Knochen dieses Abschnitts der Lamina (101) eingesetzt zu werden, und/oder ein Loch oder mehrere Löcher ist/sind, das/die es ermöglicht/ermöglichen, Schrauben in diesem Abschnitt der Lamina (101) zu platzieren.

7. Implantat (1) für die Laminoplastie nach einem der Ansprüche 1 bis 6, **dadurch gekennzeichnet, dass** die Einbaumittel (3), die von dem Zentralkörper (5) umfasst sind, zum Zusammenbauen einer oder mehrerer Transplantate (103) in der Form eines Käfigs (3) sind, der einen Querdurchlass (22) aufweist, dessen Enden seitlich austreten.

8. Implantat (1) für die Laminoplastie nach Anspruch, **dadurch gekennzeichnet, dass** der Käfig (3) eine flache dreieckige Form annimmt.

9. Implantat (1) für die Laminoplastie nach Anspruch 8, **dadurch gekennzeichnet, dass** der Käfig (3) konkave seitliche Kanten und abgerundete Ecken und, in einer Querrichtung betrachtet, eine Höhe aufweist, die auf der Seite, die dem Körper (5) gegenüberliegt, kleiner wird.

## Revendications

1. Implant (1) de laminoplastie, en particulier pour une laminoplastie cervicale, adapté à relier les deux portions (101) de la lame d'une vertèbre résultant d'une ablation de la zone centrale de cette lame, comprenant :
- un corps (5) ayant une première extrémité et une deuxième extrémité, ledit corps (5) ayant un côté externe, un côté interne, un côté supérieur et un côté inférieur ; une première direction (d1) s'étend depuis le côté extérieur vers le côté intérieur dudit corps (5) ; et
- deux parties d'ancrage (6) dont l'une est reliée à ladite première extrémité et dont l'autre est reliée à ladite deuxième extrémité, chaque partie d'ancrage (6) ayant la forme d'un U, c'est-à-dire comprenant une paroi latérale interne (15), une paroi latérale externe (16) et une paroi intermédiaire (17) reliant ladite paroi latérale interne (15) à ladite paroi latérale externe (16); une deuxième direction (d2) s'étend depuis ladite paroi intermédiaire (17), perpendiculairement à celle-ci, vers l'ouverture du U définie entre les bords d'extrémité libres de ladite paroi latérale interne (15) et ladite paroi latérale externe (16) ;
- ledit corps (5) comprend des moyens de montage (3) ;
**caractérisé en ce que** ladite paroi intermédiaire (17) de chaque partie d'ancrage (6) est située sur le côté supérieur du corps (5) de l'implant, et ladite deuxième direction (d2) est perpendiculaire à ladite première direction (d1).

2. Implant de laminoplastie (1) selon la revendication 1, **caractérisé en ce que** chaque paroi intermédiaire (17) est déformable entre une première position, dans laquelle la paroi latérale externe (16) à laquelle cette paroi intermédiaire est reliée est éloignée de la paroi latérale interne (15) à laquelle cette paroi intermédiaire est reliée, et une deuxième position, dans laquelle cette paroi latérale externe (16) est rapprochée de cette paroi latérale interne (15).

3. Implant de laminoplastie (1) selon la revendication 1 ou la revendication 2, **caractérisé en ce que** chaque paroi latérale interne (15) s'étend selon une direction formant, avec le côté interne du corps (5), un angle allant de 100 à 120°.

4. Implant de laminoplastie (1) selon la revendication 3, **caractérisé en ce que** chaque paroi latérale (6) s'étend selon une direction formant, avec le côté interne du corps (5), un angle de 110°.

5. Implant de laminoplastie (1) selon l'une quelconque des revendications 1 à 4, **caractérisé en ce que** chaque paroi latérale (15, 16) et/ou chaque paroi intermédiaire (17) comprend des moyens favorisant sa fixation par rapport à une portion de lame (101).

6. Implant de laminoplastie (1) selon la revendication 5, **caractérisé en ce que** lesdits moyens sont des dents acérées propres à être insérées dans l'os de la portion de lame (101), et/ou un ou plusieurs trous permettant la mise en place de vis au travers de cette portion de lame (101).

7. Implant de laminoplastie (1) selon l'une quelconque des revendications 1 à 6, **caractérisé en ce que** lesdits moyens de montage (3) que comprend ledit corps central (5) pour le montage d'un ou plusieurs greffons sont sous forme d'une cage (3) présentant un conduit transversal (22) dont les extrémités débouchent latéralement.

8. Implant de laminoplastie (1) selon la revendication 7, **caractérisé en ce que** la cage (3) présente une forme triangulaire aplatie.

9. Implant de laminoplastie (1) selon la revendication 8, **caractérisé en ce que** la cage (3) présente des bords latéraux concaves et des angles arrondis, et, vue dans la direction transversale, une hauteur allant en se réduisant du côté opposé audit corps (5).
